Europäisches Patentamt

European Patent Office

Office européen des brevets

(19)

(11) Publication number: **0 424 112 A2**

# EUROPEAN PATENT APPLICATION

(12)

(21) Application number: 90311356.1

(51) Int. Cl.⁵: **A61K 7/043**

(22) Date of filing: **17.10.90**

(30) Priority: **18.10.89 JP 271340/89**

(43) Date of publication of application:
**24.04.91 Bulletin 91/17**

(84) Designated Contracting States:
**DE FR GB**

(71) Applicant: **KAO CORPORATION**
**14-10, Nihonbashi Kayabacho 1-chome**
**Chuo-ku Tokyo(JP)**

(72) Inventor: **Igarashi, Tadashi**
**4-1, Kinryujico**
**Wakayama-shi, Wakayama(JP)**
Inventor: **Fukuda, Keiichi**
**10-5, Hirata 4-chome**
**Ichikawa-shi, Chiba(JP)**
Inventor: **Kondo, Akihiro**
**8-59, Nishihama 3-chome**
**Wakayama-shi, Wakayama(JP)**
Inventor: **Hosokawa, Hitoshi**
**344-1, Gyoudacho**
**Funabashi-shi, Chiba(JP)**
Inventor: **Imai, Takeo**
**2-356, Midorigaoka**
**Matsudo-shi, Chiba(JP)**

(74) Representative: **Bannerman, David Gardner et al**
**Withers & Rogers 4 Dyer's Buildings Holborn**
**London, EC1N 2JT(GB)**

(54) **Nail make-up composition.**

(57) A water-based nail make-up composition contains 5 to 60% by weight (in terms of the solids content) of an aqueous composite polymer emulsion wherein the polymer particles constituting the polymer emulsion have a multilayered structure comprising at least two layers of different chemical compositions and the outermost layer polymer has a softening temperature lower than that of the inner layer polymer.

## NAIL MAKE-UP COMPOSITION

This invention relates to a water-based nail make-up composition.

Conventional nail make-up compositions comprise nitrocellulose or alkyl resin as a film former, a plasticizer, and an organic solvent as the principal agents. However, these organic solvent-based nail make-up compositions have serious drawbacks including inflammability resulting from the use of an organic solvent, solvent odour, influences on the human body, and especially an adverse effect on the nail itself. In order to overcome these problems, water-based nail make-up compositions which do not include an organic solvent have been proposed.

For example, nail make-up compositions comprising acrylic polymer emulsions are disclosed in Japanese Patent Laid-Open Nos. 28836/1979 and 52736/1979. However, these exhibit unsatisfactory brushability, film-forming properties (especially at low temperatures), and film gloss.

Nail make-up compositions comprising an acrylic polymer microemulsion are disclosed in Japanese Patent Laid-Open Nos. 131513/1981 and 56410/1982. However, these have a drawback in that the coating film which is produced cannot withstand any mechanical rubbing.

Water-based nail make-up compositions as disclosed in Japanese Patent Laid-Open Nos. 131513/1981 and 63507/1987, are unsatisfactory since the films produced can be easily peeled off during daily use.

Under these circumstances, the inventors of this invention have made intensive studies to obtain a water-based nail make-up composition with various advantages such as excellent gloss, adhesion, water resistance and film strength without the problems of inflammability and solvent odour associated with conventional organic solvent-based compositions.

Several water-based composite polymer emulsions having a multilayered structure as used in this invention are known in the field of the paint-industry; for example, British Patent No. 928,251 describes that a multilayered structure polymer latex obtained by conducting emulsion polymerisation in three stages serves a water-based gloss paint material which can provide an excellent coating film having good water resistance, U.S. Patent No. 3,256,233 describes a water-based gloss paint comprising a polymer latex having a layered structure, formed by conducting polymerisation in two or three separate stages, and an alkali-soluble styrene/maleic anhydride alternating copolymer, and U.S. Patent No. 3,236,798 describes that an acrylonitrile/ethyl acrylate copolymer latex formed by conducting polymerisation in divided stages serves a latex paint of excellent solvent resistance. However no examples of their application to the file of cosmetics industry such as nailmake-up compositions has been found.

The present invention provides a water-based nail make-up composition characterised by containing 5 to 60% by weight (in terms of the solids content) of an aqueous composite polymer emulsion wherein the polymer particles constituting the polymer emulsion have a multilayered structure comprising at least two layers of different chemical compositions and the outermost layer polymer has a softening temperature lower than that of the inner layer polymer.

By virtue of having a multilayered structure, polymers can be used in various combinations, for example, a combination of polymers each having a high glass transition temperature and poor compatibility, which cannot be easily blended together by kneading with rolls; and the formation of a polymer in which a glass phase, a crystal phase, and a rubber phase coexist. The inventors of this invention have found that when a polymer emulsion has a softening temperature of the outermost layer lower than that of the inner layer polymer, it can exhibit good film-forming properties and high film hardness when compared with a polymer emulsion not having a multilayered structure but comprising particles having a uniform composition.

Generally known processes for producing an aqueous composite polymer emulsion comprising particles having such a multilayered structure include one conducting emulsion polymerization in seperate stages in an aqueous medium. For example, such a polymer emulsion can be produced by forming the inner and outer layers of the polymer from monomer combinations of different compositions by the so-called "seed emulsion polymerization"(see, e.g. Journal of the Japan Society of Colour Material, 50 (5), 267-275(1977). Depending on the selection of monomer combinations, the seed polymer may constitute the inner or outer layer.

Further, it is also possible by suitably selecting the monomers to design a polymer emulsion so that the polymers constituting the inner and outer layers of particles of the polymer emulsion may have different softening temperatures. Preferably, The polymer emulsion of the invention is characterized in that the polymer constituting the outermost layer has a softening temperature lower than that of the polymer constituting the inner layer. When the emulstion is applied to nails, the outermost layer polymer having a lower softening temperature forms a continuous phase and shows good film-forming properties, while the inner layer polymer having a higher softening temperature gives a suitable coating film hardness. If the polymer emulsion is designed so that the polymer constituting the outermost layer has a softening temperature higher than that of the polymer constituting the inner layer, the film-forming properties and the properties of the obtained coating film may be unsatisfactory for use as a nail make-up composition.

Although the lowest possible softening temperature of the outermost layer polymer is advantageous for film-forming properties, the coating film becomes sticky and lacks in gloss retention when it is excessively low; therefore it is preferably -10°C or higher, and more preferably, 0°C or higher. Further, it is also possible to control the film-forming properties by using a film-forming aid or a plasticizer when the film formation is difficult because of a high softening temperature of the outermost layer (for example, above room temperature).

On the other hand, it is desirable that the softening temperature of the inner layer polymer be higher than that of the outermost layer polymer by at least 10°C. When the difference between these softening temperatures is less than 10°C, the emulsion does not exhibit both of the afore-mentioned properties.

Concerning the weight ratio between the inner layer polymer and the outer layer polymer, the outermost layer polymer is preferably 0.1 to 20 parts by weight, and more preferably 0.5 to 5 parts by weight per part by weight of the inner layer polymer. When it is below 0.1 part by weight or above 20 parts by weight, the nature of the inner layer or outermost layer polymer predominates, and therefore a composite polymer emulsion effect as described earlier is not produced.

A composite polymer emulsion comprising two layers of an inner layer and an outer layer can be obtained by conducting a single seed polymerisation step. A multilayered composite polymer emulsion can be obtained repeating the seed polymerisation step. The polymer emulsion of the invention is designed so that the softening temperature of the polymer constituting the outermost layer is lower than that of the polymer constituting the inner layer.

The polymers constituting the outer layer and the inner layer in this invention can be obtained by polymerizing monomers having a polymerizable double bond. The monomers to be used are not particularly limited, and examples thereof include (meth)acrylates such as methyl acrylate, ethyl acrylate, n-butyl acrylate, lauryl acrylate, methyl methacrylate, ethyl methacrylate and n-butyl methacrylate; styrene monomers such as styrene and chlorostyrene; N-substituted (meth)acrylamides such as t-butylacrylamide; and acrylonitrile and methacrylonitrile, from which one or more members can be selected.

When the polymer constituting the inner layer is more hydrophobic than the polymer constituting the outer layer, the desired multilayered structure emulsion can be easily obtained.

Therefore, it is appropriate to select the monomers to be used in due consideration of the relative hydrophilicity/hydrophobicity balance of the obtained polymers and a difference between their softening temperatures. It is possible to prepare water-based composite polymer emulsions having a mutlilayered structure by suitably combining the above monomers and conducting polymerization according to well-known methods,such as emulsion polymerization or seed emulsion polymerization in the presence of an emulsifier or heterogenous polymerization in aqueous medium in the absence of any emulsifier.

A particularly desirable aqueous composite polymer emulsion is obtained by polymerizing one or more monomers (B), each having a polymerizable double bond,in the presence of aqueous vinyl polymer (A) obtained by adding water to a solution of an organic solvent of a copolymer, obtained by copolymerizing 0.5 to 15% by weight of a monomer having both a polymerizable double bond and an ionic or ionizable group,with 85 to 99.5% by weight of a monomer copolymerizable therewith,and distilling off the organic solvent from the mixture.

Examples of the monomers having both a polymerizable double bond and an ionic or ionizable group to be used in the production of aqueous vinyl polymer (A) of this invention include anionic, cationic and amphoteric monomers.

Examples of the anionic monomers include unsaturated carboxylic acid monomers such as acrylic acid, methacrylic acid and maleic acid and their anhydrides and salts; unsaturated sulfonic acid monomers such as styrenesulfonic acid and 2-acrylamido-2-methylpropanesulfonic acid and their salts; and unsaturated phosphorus acid monomers such as vinylphosphonic acid and acid phosphoxyethyl (mety)acrylate.

Examples of the cationic monomers include dialkylaminoalkyl (meth)acrylates or (mety)acrylamides such as N,N-dimethylaminoethyl (meth)acrylate and N,N-dimethylaminopropylacrylamide; dialkylamino styrenes such as N,N-dimethylaminostyrene and N,N-dimethylaminomethylstyrene; vinylpyridines such as 4-vinylpyridine and 2-vinylpyridine; and compounds prepared by quaternizing the above-mentioned compounds with known quaternizing agents such as an alkyl halide, a benzyl halide, an alkyl- or aryl-sulfonic acid and a dialkyl sulfate.

Examples of the amphoteric monomers include N-(3-sulfopropyl)-N-methacryloyloxyethyl-N,N-dimethylammonium betaine and N-carboxymethyl-N-methacryloyloxyethyl-N,N-dimethylammonium betaine.

Examples of the monomers having a polymerizable double bond and being copolymerizable with these monomers include (meth)acrylates such as methyl acrylate, ethyl acrylate, n-butyl acrylate, lauryl acrylate, methyl methacrylate, ethyl methacrylate and n-butyl methacrylate; styrene monomers such as styrene and chlorostyrene; N-substituted (meth)acrylamides such as t-butylacrylamide; and acrylonitrile and methacrylonitrile, from which one or more members can be selected.

In this invention, the mixing ratio of the monomer (1) having both a polymerisation double bond and a salt-forming group,to the monomer (2) copolymerisable therewith is 0.5 to 15% by weight of (1) to 85 to 99.5% by weight of (2), preferably 2 to 10% by weight of (1) to 90 to 98% by weight of (2) latter. When the amount of the monomer (1) is below 0.5% by weight, no stable aqueous vinyl polymer can be obtained, while when it is above 15% by weight, no resin having practical water resistance can be obtained.

In order to copolymerise the monomer (1) with the monomer (2), a well-known polymerisation process such as solution polymerisation, bulk polymerisation or precipitation polymerisation is conducted in the presence of well-known radical initiators. Since the polymerization system must be inverted to an aqueous phase in the subsequent step it is desirable to employ solution polymerization and to shift to the next step immediately after the polymerization. It is also possible to purify the obtained copolymer before the inversion to an aqueous phase. The average molecular weight of the copolymer is preferably 10,000 to 500,000, and more preferably 50,000 to 200,000. When it is below 10,000, the properties of the coating film are poor, while when it is above 500,000, the phase inversion is difficult to conduct, so that no aqueous vinyl polymer can be obtained.

The salt-forming groups may be either ionic or nonionic. When the salt-forming groups of the copolymer are nonionized, they are optionally ionized with a neutralizing agent. When an ionic monomer is used, it is not necessary to ionize with a neutralizing agent, but it is desirable to ionize the nonionized copolymer with a neutralizing agent because the stimulus to the nail or skin can be decreased thereby.

As the neutralizing agents, well-known acids and bases may be used according to the kinds of the salt-forming groups. Examples of the acids include inorganic acids such as hydrochloric acid and sulfuric acid; and organic acids such as acetic acid, propionic acid, lactic acid, succinic acid and glycolic acid. Examples of the bases include tertiary amines such as trimethylamine and triethylamine, ammonia and sodium hydroxide. Although the degree of neutralization is not particularly limited, it is desirable to conduct the neutralization so that the pH of the obtained aqueous vinyl polymer is in the vicinity of neutrality.

In order to invert the copolymer obtained in this way to an aqueous system, the copolymer is dissolved in an organic solvent such as an alcohol, a ketone, an ester or an ether, water is added to the solution, and the organic solvent is distilled off from the mixture.

Although the concentration of the above-mentioned organic solvent solution is suitably determined according to the composition and molecular weight of the copolymer, it is usually 10 to 80% by weight, and preferably 20 to 70% by weight.

Among the above-mentioned solvents, alcohol and/or ketone solvents are desirable because the phase inversion is easily performed with them. Although the polymerization solvent in the case of solution polymerization may be freely selected, it is desirable to use an organic solvent mentioned above to simplify the process from the polymerization to the phase inversion.

Examples of the alcohol solvent used in this invention include methanol, ethanol, n-propanol and isopropanol, among which isopropanol is desirable. Examples of the ketone solvent include acetone, methyl ethyl ketone and diethyl ketone, among which methyl ethyl ketone is preferable. These solvents may be used alone or in the form of a mixture.

The phase inversion of the above organic solvent solution to an aqueous system can be performed by conventional techniques, and it may be performed by adding water to the organic solvent solution under agitation at room temperature to 80°C, and preferably to 60°C. After the phase inversion into an aqueous system, the aqueous vinyl polymer (A) can be obtained by distilling off the organic solvent from the mixture under normal pressure or in vacuo.

Examples of the one or more monomers (B) having a polymerizable double bond, polymerized in the presence of said aqueous vinyl polymer (A) include (meth)acrylates such as methyl acrylate, ethyl acrylate, n-butyl acrylate, lauryl acrylate, methyl methacrylate, ethyl methacrylate, n-butyl methacrylate, isobutyl methacrylate and t-butyl methacrylate; styrene monomers such as styrene and chlorostyrene; N-substituted (meth)acrylamides such as t-butyl-acrylamide; and acrylonitrile and methacrylonitrile, from which one or more members can be selected.

The polymerization process may comprise adding the above-mentioned monomer (B) and a well-known radical initiator to aqueous vinyl polymer (A) and polymerizing the mixture. As the radical initiator, either a water-soluble radical initiator such as potassium persulfate, sodium persulfate or 2,2'-azobis(2-amidinopropane) dichloride,or an oil-soluble radical initiator such as 2,2'-azobis-(2,4-dimethylvaleronitrile) or 2,2'-azobisisobutyronitrile, or a combination of the two may be used. When a water-soluble initiator is used, the polymerization can be performed by the emulsion polymerization technique. When an oil-soluble initiator is used, it is added to aqueous vinyl polymer (A) after it is dissolved in a monomer solution. The polymerization temperature may be chosen bearing in mind the decomposition rate of the initiator used. The amount of monomer (B) used is preferably in such a range that the total amount of the resin component in aqueous vinyl polymer (A) and the monomer (B) is 50% by weight or below based on the entire system. When this amount is above 50% by weight, it is difficult to obtain a stable aqueous composite polymer emulsion. The amount of monomer (B) used is preferably 0.1 to 20, more preferably 0.5 to 5 times by weight of the resin component of aqueous vinyl polymer (A). These preferred ranges provide the polymer emulsion with an improvement in stability. Another preferable amount of monomer (B) used in 0.05 to 10, more preferably 0.2 to 2, times by weight the amount of resin component (A). One or more monomers (B) can be polymerized in this way without using any emulsifier in the presence of aqueous vinyl polymer (A). It is difficult to obtain a stable aqueous composite polymer emulsion, and it is desirable to add a water-soluble solvent, e.g. an alcohol solvent such as methanol or ethanol or a ketone solvent such as acetone or methyl ethyl ketone, to the system and to distill off the solvent from the mixture after polymerization. In this case, for example, monomer (B) is previously mixed with the above water-soluble solvent, and the obtained mixture is added to aqueous vinyl polymer (A).

The aqueous composite polymer emulsion obtained in this way is one having desirable film-forming properties and film strength when used as a nail make-up composition. Further, the water resistance of the obtained coating film is excellent because the emulsion does not contain any emulsifier component such as a surfactant.

Although the fine structure of the obtained aqueous composite polymer emulsion particle is not yet clear, the polymerization presumably proceeds in such a way that the aqueous vinyl polymer (A) acts as a dispersion stabilizer for liquid drops of monomer (B) and the formed polymer particles, or such that the aqueous vinyl polymer (A) acts as a seed, absorbing monomer (B). In any case, it appears that the aqueous vinyl polymer (A) in the finished polymer emulsion is situated near the surface of the particle or in the outer layer because it has an ionic group and is relatively hydrophilic, and that the (co)polymer of monomer (B) forms the core or inner layer of the particle.

Although the properties of aqueous vinyl polymer (A) and the (co)polymer of monomer (B) may be freely designed by suitably selecting monomers, a more desirable result can be attained in this invention especially when the emulsion is designed so that the softening temperature of aqueous vinyl polymer (A) may be lower than that of the (co)polymer of monomer (B). Especially, the softening temperature of the resin component of aqueous vinyl polymer (A) is preferably $-10°C$ or above, more preferably $0°C$ or above. When the softening temperature is below $-10°C$, the coating film is sticky and lacks in gloss retention. Further, in order to produce the above-mentioned effects, namely, the capability of exhibiting both of the good film-forming properties and high film hardness, the softening temperature of the (co)polymer of monomer (B) formed in the presence of aqueous vinyl polymer (A) is preferably higher than that of the resin of aqueous vinyl polymer (A) by at least $10°C$.

In this invention, the softening temperature is a value obtained by measuring the temperature at which the heat distortion of a test piece (0.1 to 0.2 mm x 3 mm x 20 mm) initiates with a thermal stress-strain measurement apparatus (TMA) (at a rate of temperature rise of $5°C/min$).

The above-mentioned aqueous composite polymer emulsion is used as a film former in the water-based nail make-up composition of this invention, and its content is 5 to 60% by weight (in terms of the solids content). When this content is lower than 5% by weight, several runs of wet-on-wet coating are necessary to obtain a coating film necessary in actual use, while when it is above 60% by weight, the viscosity of the nail make-up composition increases to lower the coatability such as brushability.

It is possible to add a pigment, a dye, an antiseptic, a perfume, optionally a plasticizer or a film-forming aid, etc., in addition to the above-mentioned aqueous composite polymer emulsion to the water-based nail make-up composition of this invention. As the plasticizers and the film-forming aids, well-known compounds such as Cellosolve, methyl Cellosolve, butyl Cellosolve, Carbitol, butyl Carbitol, Cellosolve acetate, butyl Cellosolve acetate and butyl Carbitol acetate can be used, and the amount of addition is desirably about 0 to 15% by weight from the viewpoints of the shelf stability of the aqueous composite polymer emulsion and the water resistance of the coating film.

[Examples]

Embodiments of the invention will now be described by way of example only.

All of the parts and % in these examples are by weight unless otherwise specified.

Synthesis Example 1

50 Parts of methyl ethyl ketone was fed to a reactor equipped with an agitator, reflux condenser, dropping funnel, thermometer and nitrogen inlet, and the reactor was purged with a nitrogen gas to remove dissolved oxygen.

Separately, 35 parts of methyl ethyl ketone, 56 parts of methyl methacrylate, 40 parts of n-butyl acrylate, 4 parts of acrylic acid, and 0.2 part of azobisisobutyronitrile were fed into a dropping funnel.

The mixture in the reactor was heated to 80°C under agitation, and the above methyl ethyl ketone solution of the monomer and the radical initiator was added dropwise from the dropping funnel to the mixture over a period of 2.5 hours. Two hours after the addition of the monomer, a solution of 0.2 part of azobisisobutyronitrile in 10 parts of methyl ethyl ketone was added to the mixture. After the mixture was aged at this temperature for 3 hours, a solution of 0.1 part of azobisisobutyronitrile in 5 parts of methyl ethyl ketone was added again to the mixture, and the resulting mixture was reacted for 5 hours to give a copolymer.

Part of the obtained copolymer was separated, and the determination of its molecular weight by gel permeation chromatography revealed that its weight-average molecular weight was 75,000. The calibration curve of gel permeation chromatography was prepared by using polystyrene as the standard substance (solvent: tetrahydrofuran).

The copolymer solution was cooled to room temperature and neutralized by adding 5.6 parts of triethylamine. 400 Parts of ion-exchanged water was added to the solution under agitation at 300 rpm and the solution was concentrated by distilling off the methyl ethyl ketone at 40°C in vacuo and the water at 50°C, to give an aqueous vinyl polymer of solids content of 25%.

200 Parts of this aqueous vinyl polymer and 100 parts of water were fed to a reactor equipped with an agitator, reflux condenser, dropping funnel, thermometer and nitrogen inlet, and the reactor was purged with a nitrogen gas to remove dissolved oxygen.

Separately, 50 parts of isobutyl methacrylate and 75 parts of methanol were fed into a dropping funnel.

The above methanolic monomer solution was added dropwise from the dropping funnel to the reactor under agitation over a period of 1 hour. The mixture in the reactor was heated to 70°C, and a solution of 0.2 part of potassium persulfate in 10 parts of water was added to the mixture. The mixture was aged for 6 hours at this temperature to complete the polymerization. After the mixture in the reactor was cooled to 50°C, it was concentrated by distilling off the methanol and the water at 50°C in vacuo to give an aqueous composite polymer emulsion of solids content of 35%.

Part of the obtained aqueous composite polymer emulsion was dried to separate the resin, and the softening temperature of this resin was measured with a thermal stress-strain measurement apparatus (TMA/SS-10, mfd. by Seiko Instruments, Inc.) to give two values of 23°C and 51°C. The softening temperature of a resin exclusively consisting of the above aqueous vinyl resin was separately measured to give a value of 22°C.

Synthesis Example 2

200 Parts of the aqueous vinyl resin synthesized in Synthesis Example 1 and 100 parts of water were fed to a reactor equipped with an agitator, reflux condenser, dropping funnel, thermometer and nitrogen inlet, and the reactor was purged with a nitrogen gas to remove dissolved oxygen.

Separately, 20 parts of t-butyl methacrylate, 30 parts of n-butyl methacrylate, 75 parts of ethanol and 0.2 part of 2,2'-azobis(2,4-dimethylvaleronitrile) were fed to a dropping funnel.

The above ethanolic solution of the monomer and the radical initiator was added dropwise from the dropping funnel to the mixture in the reactor under agitation over a period of 1 hour. The mixture in the reactor was heated to 60°C and aged for 6 hours at this temperature to complete the polymerization. The ethanol and the water were distilled off in the same manner as that of Synthesis Example 1 to give an aqueous composite polymer emulsion of a solids content of 35%.

The softening temperatures of the resin of the obtained aqueous composite polymer emulsion were 24°C and 55°C.

Synthesis Example 3

40 Parts of methyl methacrylate, 52 parts of ethyl acrylate and 8 parts of N,N-dimethylaminoethyl methacrylate were polymerized in methyl ethyl ketone in the same manner as that of Synthesis Example 1 to give a copolymer. The weight-average molecular weight of this copolymer was 82,000.

This copolymer was neutralized by adding thereto 4.5 parts of lactic acid and inverted into an aqueous system in the same manner as that of Synthesis Example 1 to give an aqueous vinyl resin of a solids content of 25%.

200 Parts of this resin and 100 parts of water were fed to the same reactor as that of Synthesis Example 1, and the reactor was purged with a nitrogen gas to remove dissolved oxygen.

Separately, 40 parts of methyl methacrylate was fed to a dropping funnel, and the monomer was added dropwise from the dropping funnel to the above reactor under agitation over a period of 1 hour. After the mixture in the reactor was heated to 70° C, a solution of 0.2 part of potassium persulfate in 10 parts of water was added to the mixture. The resulting mixture was aged for 6 hours at this temperature to accomplish the polymerization. The water was distilled off in the same manner as that of Synthesis Example 1 to give an aqueous composite polymer emulsion of a solids content of 35%.

The softening temperatures of the resin of the obtained aqueous composite polymer emulsion were 21° C and 98° C. The softening temperature of a resin exclusively consisting of the above aqueous vinyl resin was 20° C.

Synthesis Example 4

200 Parts of the aqueous vinyl resin synthesized in Synthesis Example 3 and 100 parts of water were fed to a reactor equipped with an agitator, reflux condenser, dropping funnel, thermometer and nitrogen inlet, and the reactor was purged with a nitrogen gas to remove dissolved gas.

Separately, 40 parts of styrene, 80 parts of ethanol and 0.2 part of 2,2′-azobis(2,4-dimethylvaleronitrile) were fed to a dropping funnel.

The above ethanolic solution of the monomer and the radical initiator was added dropwise from the dropping funnel to the mixture in the reactor under agitation over a period of 1 hour. The mixture in the reactor was heated to 60° C and aged for 6 hours at this temperature to accomplish the polymerization. The ethanol and the water were distilled off in the same manner as that of Synthesis Example 1 to give an aqueous composite polymer emulsion of a solids content of 35%.

The softening temperatures of the resin of the obtained aqueous composite polymer emulsion were 19° C and 92° C.

Examples 1 to 4

Water-based nail enamels were produced according to the following formulation. The aqueous composite polymer emulsions obtained in Synthesis Examples 1 to 4 were used in respective Examples 1 to 4.

The preparative method comprises adding a film-forming aid and a plasticizer to ion-exchanged water, dispersing a pigment in the formed solution, adding an aqueous composite polymer emulsion and other components to the dispersion, uniformly mixing the mixture by agitation and finally deaerating the resulting mixture.

The amount of each of the film-forming aid and the plasticizer used was controlled so that the film-forming temperatures of the water-based nail enamels might be substantially the same.

| Formulation | parts |
|---|---|
| aqueous composite polymer emulsion (solids content: 35%) | 100 |
| pigment (Red Pigment R-226) | 3 |
| ion-exchanged water | 10 |
| film-forming aid (Carbitol) suitable amt. | (0 - 10) |
| plasticizer (dimethyl phthalate) suitable amt. | (0 - 10) |
| perfume | 0.1 |
| antiseptic | suitable amt. |
| silicone antifoamer | suitable amt. |

Comparative Example 1

An aqueous vinyl resin of a solids content of 25% was synthesized in the same manner as that of Synthesis Example 1, and this emulsion was concentrated to give an aqueous vinyl resin of a solids content of 32%.

A water-based nail enamel was produced according to the same formulation as that of Examples 1 to 4, except that this resin was used instead of the aqueous composite polymer emulsion.

Comparative example 2

28 Parts of methyl methacrylate, 20 parts of n-butyl acrylate, 4 parts of acrylic acid and 48 parts of isobutyl methacrylate were polymerized in methyl ethyl ketone in the same manner as that of Synthesis Example 1 to give a copolymer. The weight-average molecular weight of this copolymer was 72,000.

This copolymer was neutralized by adding thereto 5.6 parts of triethylamine and inverted into an aqueous system in the same manner as that of Synthesis Example 1 to give an aqueous vinyl resin of a solids content of 30%.

A water-based nail enamel was produced in the same manner as that of Examples 1 to 4, except that this resin was used instead of the aqueous composite polymer emulsion.

The properties of the nail enamels obtained in Examples 1 to 4 and Comparative Examples 1 and 2 were evaluated by the following methods.

Table 1 gives the results.

Evaluation methods

(1) drying characteristics

The sample was applied to nails with a nail enamel brush under conditions of a temperature of 25°C and a relative humidity of 60% to measure the tack-free time.
o: 3 min or below
Δ: 3 to 6 min
x: 6 min or above

(2) gloss

The gloss of the dried coating film after 30 minutes in the evaluation of drying characteristics was evaluated with the naked eye.

(3) adhesion

The adhesion of the coating film to nails after 30 minutes in the evaluation of drying characteristics was evaluated by scraping the film from the surfaces with a microspatula.

(4) water resistance

The sample was uniformly applied to a nylon plate (0.5 mm x 15 mm x 40 mm) with a nail enamel brush, and dried for 1 hour under conditions of a temperature of 25°C and a relative humidity of 60%. The plate thus treated was immersed in water at 35°C for 1 hour to evaluate the coating film as regards deterioration (clouding, swelling, softening, peeling, etc.).

(5) abrasion resistance

The dried coating film after 30 minutes in the evaluation of drying characteristics was rubbed 50 times with a cotton cloth to observe the appearance after rubbing.

(6) odor

The odor near the mouth of a nail enamel bottle was evaluated organoleptically.
The ratings in each of the above evaluations were as follows:
◎: very good
○: good
△: average
x: poor

Table 1

| | Ex. 1 | Ex. 2 | Ex. 3 | Ex. 4 | Comp. Ex. 1 | Comp. Ex. 2 |
|---|---|---|---|---|---|---|
| drying characteristics | ○ | ○ | ○ | ○ | ○ | ○ |
| gloss | ○ | ○ | ○ | ○ | ○ | ○ |
| adhesion | ○ | ○ | ◎ | ◎ | ○ | ○ |
| water resistance | ○ | ○ | ○ | ○ | △ | △ |
| abrasion rsistance | ○ | ○ | ○ | ○ | x | x |
| odor | ○ | ○ | ◎ | ◎ | ○ | ○ |

Table 1 clearly shows that the water based nail enamel of this invention has satisfactory performances in properties necessary as a nail make-up composition.

## Claims

1. A water-based nail make-up composition characterized by containing 5 to 60% by weight (in terms of the solids content) of an aqueous composite polymer emulsion wherein the polymer particles constituting the polymer emulsion have a multilayered structure comprising at least two layers of different chemical compositions and the outermost layer polymer has a softening temperature lower than that of the inner layer polymer.

2. A water-based nail make-up composition characterized by containing 5 to 60% by weight (in terms of the solids content) of an aqueous composite polymer emulsion obtained by polymerizing one or more monomers (B) having a polymerizable double bond, in the presence of an aqueous vinyl polymer (A) obtained by adding water to a solution in an organic solvent of a copolymer obtained by copolymerizing 0.5 to 15% by weight of a monomer having a polymerizable double bond and an ionic or ionizable group,with 85 to 99.5% by weight of a monomer copolymerizable therewith and distilling off the organic solvent from the resulting mixture.

3. A water-based nail make-up composition according to claim 2, wherein the softening temperature of the polymer component of the aqueous vinyl polymer (A) is lower than that of the (co)polymer of one or more monomers (B).

4. A water-based nail make-up composition as claimed in any preceding claim in which the softening temperature of the outermost layer polymer is greater than $-10°$ C.

5. A water-based nail make up composition as claimed in any preceding claim in which the difference between the softening temperature of the outermost layer polymer and that of the inner layer polymer is at least $10°$ C.

6. A water-based nail make-up composition as claimed in claim 1 in which the weight ratio of the outer layer polymer is preferably 0.1 to 20 parts by weight per part by weight of the inner layer polymer.